# EUROPEAN PATENT APPLICATION

(11) **EP 1 987 824 A1**
(43) Date of publication of application: **05.11.2008**
(21) Application number: 07707433.4
(22) Date of filing: 25.01.2007
(51) Int. Cl.: A61K 31/09, A61K 31/122, A61P 9/00, A61P 9/12

(54) **COMPOSITION FOR NORMALIZING BLOOD PRESSURE**

(30) Priority: 25.01.2006 JP 2006016274
(71) Applicant: Kaneka Corporation, Kita-ku Osaka-shi, Osaka 530-8288 (JP)
(72) Inventor: IKEHARA, Toshinori, Takasago-shi Hyogo 676-0011 (JP); FUJII, Kenji, Kobe-shi Hyogo 651-1202 (JP); TEMMARU, Kiyoshi, Osaka-shi Osaka 535-0003 (JP); KITAHARA, Mikio, Kobe-shi Hyogo 654-0067 (JP); FUKUTOMI, Naoki, Suita-shi Osaka 565-0861 (JP)
(74) Representative: Gillet, Raphaëlle
(86) International application number: PCT/JP2007/051201
(87) International publication number: WO 2007/086480

(57) **Abstract**

The present invention aims to provide a composition suitable for normalizing the blood pressure, which is useful as food, health food, supplement, nutritional supplement, food for specified health uses, food with nutrient function claims, pharmaceutical product, quasi-drug, feed or pet food.

The present inventors have found that coenzyme Q has a superior effect for normalizing the blood pressure and completed the present invention. That is, the present invention provides a composition for normalizing the blood pressure, which contains reduced coenzyme Q as an active ingredient, and a composition for normalizing the blood pressure, which contains oxidized coenzyme Q as an active ingredient and improves hypotension.

## Description

### Technical Field

The present invention relates to a composition suitable for normalizing the blood pressure. That is, this invention relates to a safe composition permitting long-term ingestion, which can reduce various unidentified complaints caused by hypotension, such as dizziness, feeling of coldness, difficulty in awakening in the morning, lightheadedness, headache and the like, in subjects with lower blood pressure, and can promote normalization of the blood pressure in subjects with higher blood pressure. In addition, the invention relates to a method of normalizing the blood pressure, which comprises a step of administering the composition to the above-mentioned subjects.

### Background Art

According to the report of the Joint National Committee published in 1993, the optimal range of systolic blood pressure is not more than 120 mmHg, where a systolic blood pressure of 120 - 139 mmHg is classified as normal blood pressure and a systolic blood pressure of not less than 140 mmHg is classified as hypertension. In general, hypertension is a disease regarded as a risk factor causing various diseases, and actively studied in pursuit of a treatment and prophylaxis thereof. Its harm on the health as one of the metabolic syndromes has increasingly been clarified in recent years.

On the other hand, hypotension is suggested to be a cause of various symptoms such as dizziness while standing, lightheadedness and the like. From clinical aspects, however, hypotension is not considered a problematic disease requiring treatments. As such, there is no clear classification of hypotension; however, a systolic blood pressure of 110 mmHg or below is generally considered hypotension. Recently, various symptoms caused by hypotension mostly in young women, i.e., unidentified complaints, resulting from changes of lifestyle habits, physical stress by dieting and the like, are becoming serious problems. While hypertension does not generally show clear clinical symptoms, various symptoms caused by hypotension pose problems in everyday life, reduce quality of life (QOL) and increase burden on life. In addition, hypertension, admitted as a disease, affects the life itself. In view of the above, normal blood pressure is expected not only to remove an adverse influence on life, but also bring a healthy life by improving QOL.

To improve hypotension, a drug therapy using a sympathomimetic agent to elevate the blood pressure is mainly employed. Being a pharmaceutical product, however, use of the drug is limited and the basic problem has not been solved.

In such situation, it has been proposed to improve these symptoms with a food easy to take every day, such as supplements and the like. For example, patent reference 1 describes that an extract of Musaceae fruit has a blood pressure-elevating effect. However, the safety of ingestion thereof as a general food has not been sufficiently established, since its effect on the blood pressure in the normal range or above is not clear. Moreover, no description is provided as to the species difference in the elevation of blood pressure, and the actual effect on human is completely unknown.

In addition, patent reference 2 proposes an agent for the prophylaxis or treatment of hypotension, which comprises ferulic acids. The reference describes a blood pressure decrease-suppressive action on hemorrhage rats, a blood pressure elevation-suppressive action on spontaneously hypertensive rats, and inaction on normal blood pressure rats. However, clear description is not found as to a low pressure-elevating action, and the effect on human is unknown. As such, the reference does not entirely provide sufficient evidence, and practicability thereof is not clear.

As mentioned above, since a blood pressure out of the normal range causes specific abnormality in the physical symptoms, it is expected that a healthy life can be led and the quality of life (i.e., QOL) can be improved by maintaining the normal blood pressure. However, a composition suitable for the object, which is safe, permits long-term ingestion and provides expected effect, has not been developed yet.

As coenzyme Q, coenzyme Q₁ to coenzyme Q₁₃ are known based on the repeat structure of the side chain. Coenzyme Q₁₀ is the main coenzyme Q for mammals, and coenzyme Q₁₀ is used for human. Coenzyme Q₁₀ is localized in mitochondria, lysosome, Golgi body, microsome, peroxisome, cellular membrane and the like, and is an essential substance for the functional maintenance of living organisms, since it is involved in the activation of ATP production, antioxidant action in living body, and membrane stabilization, as a constituent component of the electron transport system. Coenzyme Q₁₀ is known to include oxidized type and reduced type, and the oxidized type is named ubiquinone and the reduced type is named ubiquinol. In the electron transport system, coenzyme Q is known to transmit electrons by repeating oxidation and reduction. In addition, the reduced type is considered the main type since an antioxidant activity is shown only by the reduced type, and coenzyme Q₁₀ in living body mostly exists as the reduced type. However, because reduced coenzyme Q₁₀ has problem with oxidation stability, oxidized coenzyme Q₁₀ alone has been used industrially. For such reason, generally, an indication of coenzyme Q₁₀ means oxidized coenzyme R₁₀, and when the reduced type is meant, it is indicated as ubiquinol or reduced coenzyme Q₁₀.

The oxidized coenzyme Q₁₀ has been conventionally used as an auxiliary agent for congestive heart failure. However, it is recently wide-used as supplement all over the world. The physiological activity thereof is widely studied, and many physiological activities are known such as antidiabetic activity (non-patent reference 1), antifatigue activity (patent references 3 - 5), anti-atherogenic activity (non-patent reference 2) and the like. In addition, a blood flow improving agent comprising proanthocyanidin and an antioxidant as active ingredients is disclosed, where coenzyme Q is recited as one of the antioxidants. However, the effect of coenzyme Q on the blood pressure is not described at all (patent reference 6). Only a weak blood pressure-lowering effect of oxidized coenzyme Q on hypertensive patients is known at most (non-patent references 1, 3, 4), and no report is found on a blood pressure-elevating effect for hypotension. The reduced coenzyme Q₁₀ is known to show an antidiabetic effect (patent reference 7), an ulcerative colitis treatment effect (patent reference 8) and the like.
patent reference 1: JP-A-6-157328
patent reference 2: JP-A-2002-145766
patent reference 3: JP-A-7-330584
patent reference 4: JP-A-7-330593
patent reference 5: JP-A-10-287560
patent reference 6: JP-A-2005-123707
patent reference 7: JP-A-2003-877895
patent reference 8: JP-A-2003-095931
non-patent reference 1: European J. of Clinical Nutrition, 2002, Vol. 56, pp. 1137-1142
non-patent reference 2: Molecular and Cellular Biochemistry, 2003, Vol. 246, pp. 75-82
non-patent reference 3: Biomedical and Clinical Aspects of Coenzyme Q, 1986, Vol. 5, pp. 337-343
non-patent reference 4: Folia pharmacologica Japonica, 1972, Vol. 68, pp. 460-472

### Disclosure of the Invention

### Problems to be Solved by the Invention

The present invention aims to provide a composition suitable for normalizing the blood pressure, which is useful as food, health food, supplement, nutritional supplement, food for specified health uses, food with nutrient function claims, pharmaceutical product or quasi-drug. **Means of Solving the Problems**

The present inventors have conducted intensive studies in view of the above-mentioned situation and found that both oxidized coenzyme Q and reduced coenzyme Q have a superior blood pressure-elevating action on hypotension, and reduced coenzyme Q shows a blood pressure-lowering action on slightly higher blood pressure, which resulted in the completion of the present invention.

Accordingly, the present invention provides the following.
[1] A composition for normalizing the blood pressure, comprising reduced coenzyme Q represented by the following formula (1):

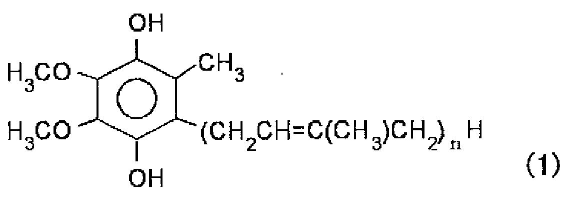

wherein n is an integer of 1 - 12, as an active ingredient. [2] The composition of [1], further comprising oxidized coenzyme Q represented by the following formula (2):

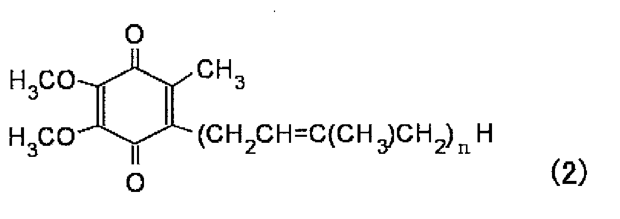

wherein n is an integer of 1 - 12.
[3] The composition of [1] or [2], which improves hypertension.
[4] The composition of [1] or [2], which improves hypotension.
[5] A composition for normalizing the blood pressure, comprising oxidized coenzyme Q represented by the following formula (2):

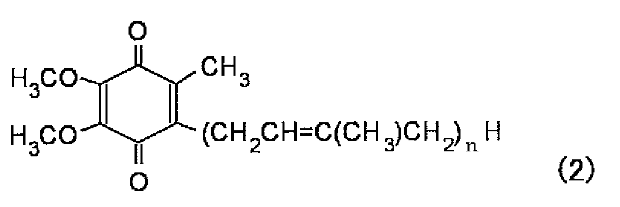

wherein n is an integer of 1 - 12, as an active ingredient, which improves hypotension.
[6] The composition of any one of [1] to [4], wherein the reduced coenzyme Q is a reduced coenzyme Q₁₀ wherein n is 10.
[7] The composition of any one of [2] to [5], wherein the oxidized coenzyme Q is an oxidized coenzyme Q₁₀ wherein n is 10. [8] The composition of any one of [1] to [7], comprising an antioxidant substance and/or an antioxidant enzyme.
[9] The composition of [8], wherein the antioxidant substance is at least one kind selected from the group consisting of vitamin E, vitamin E derivative, vitamin C, vitamin C derivative, probucol, lycopene, vitamin A, carotenoids, vitamin B, vitamin B derivative, flavonoids, polyphenols, glutathione, α-lipoic acid and selenium.
[10] The composition of [8], wherein the antioxidant enzyme is at least one kind selected from the group consisting of superoxide dismutase (SOD), glutathione peroxidase, glutathione-S-transferase, glutathionereductase, catalase and ascorbic acid peroxidase.
[11] The composition of any one of [1] to [8], comprising a nutritious supplement component.
[12] The composition of [11], wherein the nutritious supplement component is at least one kind selected from the group consisting of amino acid, metal ion, saccharides, proteins, fatty acids and vitamins.
[13] The composition of any one of [1] to [12], which is a drink or food.
[14] The composition of any one of [1] to [12], which is an oral pharmaceutical product.
[15] The composition of any one of [1] to [12], which is a feed or pet food.
[16] A method of normalizing the blood pressure, which comprises administering a composition comprising reduced coenzyme Q represented by the following formula (1):

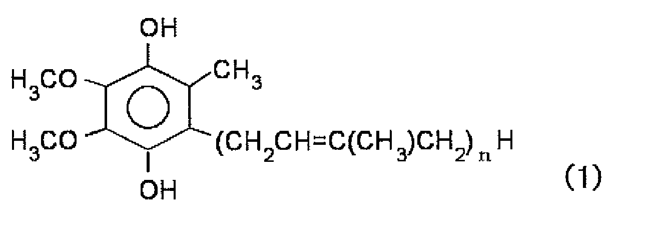

wherein n is an integer of 1 - 12, as an active ingredient, to a subject of administration.
[17] The method of normalizing the blood pressure, which comprises administering the composition of [16] further comprising oxidized coenzyme Q represented by the following formula (2):

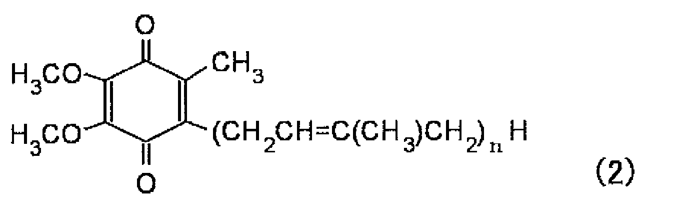

wherein n is an integer of 1 - 12, to a subject of administration.
[18] The method of [16] or [17], wherein the normalization of blood pressure is an improvement of hypertension.
[19] The method of [16] or [17], wherein the normalization of blood pressure is an improvement of hypotension.
[20] A method of improving hypotension, which comprises administering a composition comprising oxidized coenzyme Q represented by the following formula (2):

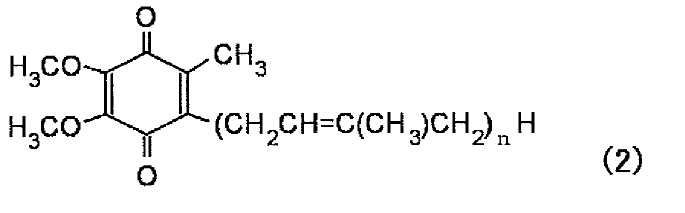

wherein n is an integer of 1 - 12, as an active ingredient, to a subject of administration.
[21] Use of reduced coenzyme Q represented by the following formula (1):

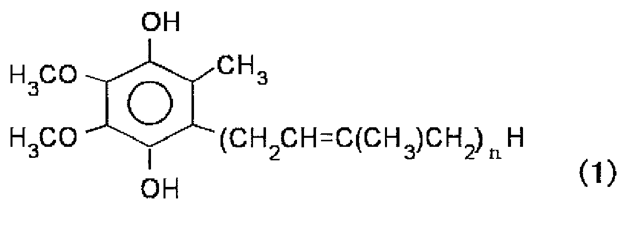

wherein n is an integer of 1 - 12, for the production of a composition for normalizing the blood pressure.
[22] The use of [21], further comprising use of oxidized coenzyme Q represented by the following formula (2):

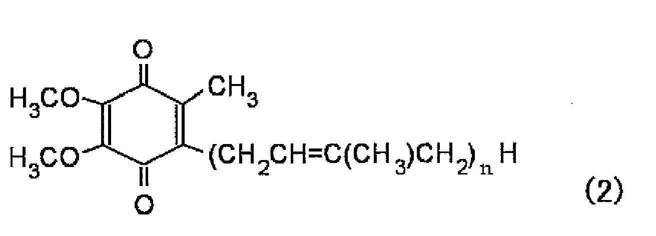

wherein n is an integer of 1 - 12.
[23] The use of [21] or [22], wherein the blood pressure normalizing composition is a composition for improving hypertension.
[24] The use of [21] or [22], wherein the blood pressure normalizing composition is a composition for improving hypotension.
[25] Use of oxidized coenzyme Q represented by the following formula (2):

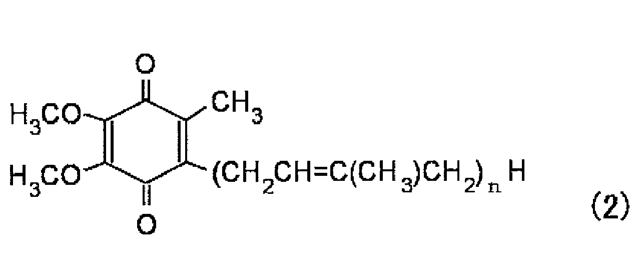

wherein n is an integer of 1 - 12, for the production of a composition for improving hypotension.
[26] A commercial package comprising the composition of any one of [1] to [15] and a written matter relating to the composition, which states that the composition can or should be used for normalizing the blood pressure.
[27] The composition of [1] to [5], which is a food.
[28] The composition of [27], wherein the food is a food with health claims.
[29] The composition of [28], wherein the food with health claims is a food for specified health uses.
[30] The composition of [1] to [5], which is a pharmaceutical product.

### Effect of the Invention

According to the present invention, food, health food, nutritional supplement, supplement, pharmaceutical product, quasi-drug, feed, pet food and the like, which have superior normalizing action on the blood pressure and are easy to take every day, can be obtained.

### Best Mode for Carrying out the Invention

The present invention is explained in detail in the following.

A first composition for normalizing the blood pressure of the present invention contains reduced coenzyme Q as an active ingredient. The coenzyme Q may be reduced coenzyme Q alone or a mixture of reduced coenzyme Q and oxidized coenzyme Q. In this case, the proportion of reduced coenzyme Q relative to the total amount of coenzyme Q can be appropriately determined according to the concept of the product thereof and the like. An extremely increased proportion of reduced coenzyme Q may increase the cost due to a stabilization measure therefor and the like. However, a higher normalization effect on the blood pressure can be expected. For example, to improve hypertension, the ratio of reduced coenzyme Q relative to the total amount of coenzyme Q (i.e., reduced coenzyme Q:oxidized coenzyme Q) is generally 100:0 - 0:100, preferably 99:1 - 1:99, more preferably 98:2 - 20:80, particularly preferably 98:2 - 40:60. In addition, to improve hypotension, the ratio is generally 100:0 - 0:100, preferably 99:1 - 1:99.

The first composition for normalizing the blood pressure of the present invention has an effect to improve hypertension and an effect to improve hypotension. In other words, it has an effect to return the blood pressure outside the normal range to normal range.

A second composition for normalizing the blood pressure of the present invention contains oxidized coenzyme Q as an active ingredient, and has an effect to improve hypotension. The oxidized coenzyme Q to be used for the composition for normalizing the blood pressure of the present invention is represented by the above-mentioned formula (2). Of them, oxidized coenzyme Q₁₀ wherein n is 10 is preferably used.

In the present invention, as oxidized coenzyme Q, one obtained by a conventionally known method, such as fermentation method, synthesis method and extraction from fauna and flora can be utilized. Particularly, one having an all trans structure, which is obtained by a method other than synthesis method, such as fermentation method and the like, is preferable from the aspect of safety, and can be exemplified by Kaneka Coenzyme Q10 (manufactured by Kaneka Corporation).

The reduced coenzyme Q to be used for the blood pressure normalizing composition of the present invention is represented by the above-mentioned formula (1). Particularly, a reduced coenzyme Q₁₀, wherein n is 10, is preferably used. In the present invention, the method for obtaining reduced coenzyme Q is not particularly limited and, for example, a method including producing coenzyme Q, which is a mixture of oxidized form and reduced form, by a conventional method, and concentrating a reduced coenzyme Q segment in an eluent by chromatography and the like can be employed. In this case, it is possible to add a general reducing agent such as sodium borohydride, sodium dithionite (sodium hydrosulfite) and the like as necessary to the above-mentioned coenzyme Q, reduce oxidized coenzyme Q contained in the above-mentioned coenzyme Q by a conventional method to give reduced coenzyme Q, and concentrate the reduced coenzyme Q by chromatography. In addition, reduced coenzyme Q can also be obtained by a method including reacting existing high purity coenzyme Q with the above-mentioned reducing agent. Alternatively, fungus body containing reduced coenzyme Q and the like can be used. Alternatively, oxidized coenzyme Q can be reduced to reduced coenzyme Q in a preparation by formulating the preparation of oxidized coenzyme Q together with a substance having a reducing action such as vitamins and the like.

A simple indication of coenzyme Q in the following specification means any of oxidized coenzyme Q, reduced coenzyme Q and a mixture of oxidized coenzyme Q and reduced coenzyme Q.

The proportion of reduced form in coenzyme Q is generally determined by a method including quantifying oxidized coenzyme Q and reduced coenzyme Q in a sample by an HPLC system using a UV detector and calculating the proportion based on the obtained amount ratio, or a method including calculating the proportion of oxidized coenzyme Q and reduced coenzyme Q from peak areas obtained by a system combining HPLC with an electrochemical detector. A system incorporating an electrochemical detector is highly useful for measuring the ratio of reduced type present in a trace amount in a living organism or sample, since it can specifically measure an oxidized or reduced substance and has high sensitivity. All proportions of reduced coenzyme Q shown in the present invention were quantified by an HPLC system incorporating an electrochemical detector.

In the blood pressure normalizing composition of the present invention, coenzyme Q may be directly ingested as a single composition. However, since coenzyme Q is liposoluble, it is preferably ingested in the form of a dispersion or solution in general edible fats and oils. Such edible fats and oils is not particularly limited and, for example, vegetable oils such as corn oil, rape seed oil, high erucic acid rapeseed oil, soybean oil, sesame oil, olive oil, safflower oil, cottonseed oil, sunflower oil, rice germ oil, perilla oil, perilla oil, flaxseed oil, evening primrose oil, cacao butter, peanuts oil, palm oil, palm kernel oil and the like, animal oils such as fish oil, beef fat, lard, milk fat, egg-yolk oil and the like, synthetic oils such as medium-chain triglyceride and the like, or fats and oils obtained by fractionation, hydrogenation, transesterification and the like of these as starting materials, or a mixed oil thereof can be used. Alternatively, coenzyme Q can be ingested in a form after processing by a known technique, such as inclusion compound of cyclodextrin or oil-in-water emulsion.

The blood pressure normalizing composition of the present invention may further contain as appropriate, besides the above-mentioned coenzyme Q, other material acceptable as a pharmaceutical or food, by mixing according to a conventional method. Examples of the material include excipient, disintegrant, lubricant, binder, antioxidant, colorant, anticoagulant, absorption promoter, solubilizing agent, stabilizer and the like.

The above-mentioned excipient is not particularly limited and, for example, sucrose, lactose, glucose, cornstarch, mannitol, crystalline cellulose, calcium phosphate, calcium sulfate and the like can be mentioned.

The above-mentioned disintegrant is not particularly limited and, for example, starch, agar, calcium citrate, calcium carbonate, sodium hydrogen carbonate, dextrin, crystalline cellulose, carboxymethylcellulose, tragacanth and the like can be mentioned.

The above-mentioned lubricant is not particularly limited and, for example, talc, magnesium stearate, polyethylene glycol, silica, hydrogenated vegetable oil and the like can be mentioned.

The above-mentioned binder is not particularly limited and, for example, ethylcellulose, methylcellulose, hydroxypropylmethylcellulose, tragacanth, shellac, gelatin, gum arabic, polyvinylpyrrolidone, polyvinyl alcohol, polyacrylic acid, polymethacrylic acid, sorbitol and the like can be mentioned.

The above-mentioned antioxidant is not particularly limited and, for example, ascorbic acid, tocopherol, vitamin A, β-carotene, sodium bisulfite, thiosodium sulfate, pyrrosodium sulfite, citric acid and the like can be mentioned.

The above-mentioned colorant is not particularly limited and, for example, those permitted for addition to food and pharmaceutical products, and the like can be used.

The above-mentioned anticoagulant is not particularly limited and, for example, stearic acid, talc, light anhydrous silicic acid, hydrated silicon dioxide and the like can be mentioned.

The above-mentioned absorption promoter is not particularly limited and, for example, surfactants such as higher alcohols, higher fatty acids, glycerolfatty acid ester and the like can be mentioned.

The above-mentioned solubilizing agents are not particularly limited and, for example, organic acids such as fumaric acid, succinic acid, malic acid and the like can be mentioned.

The above-mentioned stabilizer is not particularly limited and, for example, benzoic acid, sodium benzoate, ethyl parahydroxybenzoate and the like can be mentioned.

The blood pressure normalizing composition of the present invention can concurrently contain an antioxidant substance or antioxidant enzyme. The antioxidant substance is not particularly limited and, for example, vitamin E, vitamin E derivative, vitamin C, vitamin C derivative, probucol, lycopene, vitamin A, carotenoids, vitamin B, vitamin B derivative, flavonoids, polyphenols, glutathione, α-lipoic acid, selenium and the like can be mentioned. The proportion of coenzyme Q and antioxidant substance is generally 100:1 - 1:100, preferably 10:1 - 1:10.

In addition, the antioxidant enzyme is not particularly limited and, for example, superoxide dismutase (SOD), glutathione peroxidase, glutathione-S-transferase, glutathione reductase, catalase, ascorbic acid peroxidase and the like can be mentioned. The proportion of coenzyme Q and antioxidant enzyme is generally 100:1 - 1:100, preferably 10:1 - 1:10. These antioxidant substance and antioxidant enzyme may be used alone or as a mixture of two or more kinds thereof.

The blood pressure normalizing composition of the present invention can also contain other nutritional fortification components. The nutritional fortification component is not particularly limited and, for example, creatine, taurine, vitamin B₁, vitamin B derivative, amino acid and a mixture of these substances can be mentioned. The proportion of coenzyme Q and the nutritional fortification component is generally 100:1 - 1:100, preferably 10:1 - 1:10.

The blood pressure normalizing composition of the present invention can also contain a nutritious supplement component. The nutritious supplement component is not particularly limited and, for example, amino acid, metal ion, saccharides, proteins, fatty acids, vitamin and the like can be mentioned. The proportion of coenzyme Q and the nutritious supplement component is generally 100:1 - 1:100, preferably 10:1 - 1:10.

The blood pressure normalizing composition of the present invention can take the form of a drink or food (food composition). When the blood pressure normalizing composition of the present invention is a general food, its form is not particularly limited and, for example, edible fat and oil composition, cooking oil, spray oil, butter, margarine, shortening, whipping cream, concentrated milk, whiteners, dressings, pickle liquids, breads, cakes, pies, cookies, Japanese confectionaries, snacks, fried snacks, chocolates and chocolate confectionaries, rice confectionaries, roux, sauce, basting, toppings, ice creams, noodles, bread mix, fried food, processed meat products, fish paste products, frozen food such as frozen entrees, frozen meat and frozen vegetables, rice, jam, cheese, cheese food, cheese-like food, chewing gums, candies, fermented milk, canned food, drinks and the like can be mentioned.

In addition, such foods can be provided as food with health claims or dietary supplement. The food with health claims also includes food and drink, particularly food for specified health uses or food with nutrient function claims and the like, with an indication that they are used for normalizing the blood pressure.

The blood pressure normalizing composition of the present invention can be used for drugs, as an agent for normalizing the blood pressure, and can be particularly formed into an oral pharmaceutical product. While the form of the blood pressure normalizing composition of the present invention as an oral pharmaceutical product is not particularly limited, powder, capsule, soft capsule, tablet and the like can be mentioned. In addition, the blood pressure normalizing composition of the present invention in such dosage form can also be used as a supplement, a quasi-drug and the like, rather than a pharmaceutical product.

The effective ingestion dose of coenzyme Q of the present invention for an adult per day to normalize the blood pressure is 10 - 500 mg, preferably 30 - 300 mg, more preferably 50 - 200 mg, as reduced coenzyme Q₁₀. More particularly, the effective ingestion dose of reduced coenzyme Q₁₀ per day to improve hypertension is 10 - 500 mg, preferably 30 - 500 mg, more preferably 50 - 300 mg. In addition, the effective ingestion dose of reduced coenzyme Q₁₀ per day to improve hypotension is 10 - 400 mg, preferably 30 - 250 mg, more preferably 50 - 200 mg. When the ingestion dose of reduced coenzyme Q₁₀ is less than 10 mg, a sufficient blood pressure-elevating effect and a sufficient blood pressure-lowering effect may not be observed.

Similarly, the effective ingestion dose per day of oxidized coenzyme Q₁₀ to normalize the blood pressure is 50 - 2000 mg, preferably 200 - 1200 mg, more preferably 500 - 900 mg. When the ingestion dosage is less than 50 mg, a sufficient blood pressure-elevating effect may not be provided.

The effective ingestion doses of reduced coenzyme Q₁₀ and oxidized coenzyme Q10 to be used in combination cannot be defined generally since they vary depending on the content ratio of the reduced coenzyme Q₁₀ and oxidized coenzyme Q10. However, since enhanced absorption can be expected by combining them, the effective ingestion dose is 10 - 450 mg, preferably 20 - 300 mg, more preferably 30 - 200 mg.
However, the above-mentioned ingestion doses are known to vary depending on the dosage form of the preparation, and a highly absorbable preparation can achieve a given object even with a smaller dosage.

The above-mentioned daily dose can be ingested at once or in several times (e.g., 3 times). Particularly, when the object is correction of hypotension, better normalized blood pressure can be expected in the early morning by ingestion after dinner once a day. However, the method of use is not limited thereto.

In the present invention, the above-mentioned single ingestion dose can be packaged as 1 unit. For example, when the food is what is called a health food or a pharmaceutical agent, a form wherein the above-mentioned amount is packaged as a unit dose for single ingestion and the like can be mentioned. For example, when the food is a health drink, a form wherein the above-mentioned amount is suspended or dissolved to give a drink, which is packed in a bottle etc. for a single consumption and the like can be mentioned.

The blood pressure normalizing composition of the present invention can be provided as feed, pet food and the like. Examples of the target thereof include mammals (e.g., mouse, rat, hamster, rabbit, cat, dog, bovine, sheep, monkey etc.)

The present invention provides a method of normalizing the blood pressure, which comprises administering a composition containing reduced coenzyme Q and/or oxidized coenzyme Q as an active ingredient to a subject of administration whose blood pressure is outside the normal range. The method of normalizing the blood pressure aims at bringing the blood pressure outside the normal range back to fall within the normal range, and includes a method of improving hypertension and a method of improving hypotension. In addition, the present invention provides a method of improving hypotension, which comprises administering a composition containing oxidized coenzyme Q as an active ingredient to the subject of administration.

When reduced coenzyme Q and oxidized coenzyme Q are to be administered (ingested) and they can be combined at the time of administration, reduced coenzyme Q and oxidized coenzyme Q may be simultaneously formulated and administered as a single preparation. Alternatively, they may be separately formulated and the resulting two kinds of preparations may be combined and administered simultaneously or at a time interval.

The present invention further includes a commercial package comprising the composition of the present invention and a written matter relating to the composition, which states that the composition can or should be used for normalizing the blood pressure (e.g., an instruction sheet for performing the above-mentioned method by using a kit for practicing the above-mentioned method).

### Examples

The present invention is explained in more detail in the following by referring to Examples, which are not to be construed as limitative.

### (Example 1) Evaluation of blood pressure normalization by oxidized coenzyme Q₁₀ in healthy subjects

Healthy male volunteers (22 subjects) ingested soft capsule preparation containing oxidized coenzyme Q₁₀ (150 mg) by 6 capsules (total 900 mg) per day, and the blood pressure before breakfast was measured before and after ingestion for 4 weeks. After the measurement, the subjects were divided into three groups of hypotension group (n=6, not more than 110 mmHg), normal blood pressure group (n=8, 110 - 130 mmHg) and mild hypertension group (n=8, 130 - 150 mmHg), according to the systolic blood pressure before ingestion, and analyzed. The results are shown in Table 1. In the hypotension group, a significant increase in the blood pressure was observed after ingestion of oxidized coenzyme Q₁₀ as compared to before ingestion, and the blood pressure was restored to the normal level. The normal blood pressure group and mild hypertension group did not show significant variations. Consequently, a hypotension improving effect of oxidized coenzyme Q₁₀ in healthy human subjects has been clarified.

**[Table 1]**

| Effect of oxidized coenzyme Q₁₀ on diastolic blood pressure | | |
|---|---|---|
| | diastolic blood pressure (mmHg) | |
| | before ingestion | 4 weeks after ingestion |
| hypotension | 105.5±4.4 (100) | 126.5±2.9 (120**) |
| normal blood pressure | 126.1±2.7 (100) | 127.6±7.7 (101) |
| mild hypertension | 135.8±4.6 (100) | 134.8±9.4 (100) |

| | | |
|---|---|---|
| **p<0.01 U-test | | |

### (Example 2) Evaluation of blood pressure normalization by reduced coenzyme Q₁₀ in healthy subjects

Healthy male and female volunteers (20 subjects) ingested 5 soft capsules (total 150 mg) per day each containing 30 mg of reduced coenzyme Q₁₀ (containing about 2% of oxidized coenzyme Q₁₀), and the blood pressure before breakfast was measured before and after ingestion for 4 weeks. After the measurement, the subjects were divided into three groups of hypotension group (n=8, not more than 110 mmHg), normal blood pressure group (n=8, 110 - 130 mmHg) and mild hypertension group (n=4, 130 - 150 mmHg), according to the systolic blood pressure before ingestion, and analyzed. The results are shown in Table 2. In the hypotension group, a significant increase in the blood pressure was observed after ingestion of reduced coenzyme Q₁₀ as compared to before ingestion, and the blood pressure was restored to the normal level. The normal blood pressure group did not show significant variations. In contrast, the mild hypertension group showed a significant decrease in the blood pressure and restored the normal blood pressure.

**[Table 2]**

| Effect of reduced coenzyme Q₁₀ on diastolic blood pressure | | |
|---|---|---|
| | diastolic blood pressure (mmHg) | |
| | before ingestion | 4 weeks after ingestion |
| hypotension | 99.6±4.9 (100) | 117.5±6.2 (118**) |
| normal blood pressure | 125.3±3.1 (100) | 125.5±9.1 (100) |
| mild hypertension | 140.5±9.2 (100) | 125.1±6.1 (89*) |

| | | |
|---|---|---|
| *p<0.05, **p<0.01 U-test | | |

Consequently, the blood pressure normalizing effect of reduced coenzyme Q₁₀, that is, increasing the blood pressure for hypotension and lowering the blood pressure for hypertension to restore the normal blood pressure, was observed in healthy subjects.

### (Formulation Example 1) (powder)

The oxidized coenzyme Q₁₀ was dissolved in ethanol, adsorbed onto microcrystalline cellulose and dried under reduced pressure. This was mixed with cornstarch under a nitrogen stream to give a powder.

| | |
|---|---|
| oxidized coenzyme Q₁₀ | 10 parts by weight |
| microcrystalline cellulose | 40 parts by weight |
| cornstarch | 55 parts by weight |

### (Formulation Example 2) (powder)

The reduced coenzyme Q₁₀ was dissolved in ethanol, adsorbed onto microcrystalline cellulose and dried under reduced pressure. This was mixed with cornstarch under a nitrogen stream to give a powder.

| | |
|---|---|
| reduced coenzyme Q₁₀ | 10 parts by weight |
| microcrystalline cellulose | 40 parts by weight |
| cornstarch | 55 parts by weight |

### (Formulation Example 3) (powder)

The oxidized coenzyme Q₁₀ and the reduced coenzyme Q₁₀ were dissolved in ethanol, adsorbed onto microcrystalline cellulose and dried under reduced pressure. This was mixed with cornstarch under a nitrogen stream to give a powder.

| | |
|---|---|
| reduced coenzyme Q₁₀ | 9.8 parts by weight |
| oxidized coenzyme Q₁₀ | 0.2 part by weight |
| microcrystalline cellulose | 40 parts by weight |
| cornstarch | 55 parts by weight |

### (Formulation Example 4) (capsule)

A powder having the following formulation was prepared in the same manner as in Formulation Example 1, and packed in a gelatin capsule by a conventional method. The capsule was sealed, packed under a nitrogen atmosphere and refrigerated.

| | |
|---|---|
| oxidized coenzyme Q₁₀ | 20 parts by weight |
| microcrystalline cellulose | 40 parts by weight |
| cornstarch | 20 parts by weight |
| lactose | 65 parts by weight |
| magnesium stearate | 3 parts by weight |
| polyvinylpyrrolidone | 2 parts by weight |

### (Formulation Example 5) (capsule)

A powder having the following formulation was prepared in the same manner as in Formulation Example 2, and packed in a gelatin capsule by a conventional method. The capsule was sealed, packed under a nitrogen atmosphere and refrigerated.

| | |
|---|---|
| reduced coenzyme Q₁₀ | 20 parts by weight |
| microcrystalline cellulose | 40 parts by weight |
| cornstarch | 20 parts by weight |
| lactose | 65 parts by weight |
| magnesium stearate | 3 parts by weight |
| polyvinylpyrrolidone | 2 parts by weight |

### (Formulation Example 6) (capsule)

A powder having the following formulation was prepared in the same manner as in Formulation Example 3, and packed in a gelatin capsule by a conventional method. The capsule was sealed, packed under a nitrogen atmosphere and refrigerated.

| | |
|---|---|
| reduced coenzyme Q₁₀ | 20 parts by weight |
| oxidized coenzyme Q₁₀ | 0.4 part by weight |
| microcrystalline cellulose | 40 parts by weight |
| cornstarch | 20 parts by weight |
| lactose | 65 parts by weight |
| magnesium stearate | 3 parts by weight |
| polyvinylpyrrolidone | 2 parts by weight |

### (Formulation Example 7) (soft capsule)

Corn oil was heated to 50°C and oxidized coenzyme Q₁₀ melted at the same temperature and vitamin E were added and dissolved therein. This was processed into a soft capsule by a conventional method.

| | |
|---|---|
| oxidized coenzyme Q₁₀ | 50 parts by weight |
| corn oil | 300 parts by weight |
| vitamin E | 120 parts by weight |

### (Formulation Example 8) (soft capsule)

Corn oil was heated to 50°C and reduced coenzyme Q₁₀ melted at the same temperature and vitamin E were added and dissolved therein. This was processed into a soft capsule by a conventional method.

| | |
|---|---|
| reduced coenzyme Q₁₀ | 50 parts by weight |
| corn oil | 300 parts by weight |
| vitamin E | 120 parts by weight |

### (Formulation Example 9) (soft capsule)

Corn oil was heated to 50°C, and reduced coenzyme Q₁₀ and oxidized coenzyme Q₁₀ melted at the same temperature and vitamin E were added and dissolved therein. This was processed into a soft capsule by a conventional method.

| | |
|---|---|
| reduced coenzyme Q₁₀ | 49 parts by weight |
| oxidized coenzyme Q₁₀ | 1 part by weight |
| corn oil | 300 parts by weight |
| vitamin E | 120 parts by weight |

### (Formulation Example 10) (tablet)

Oxidized coenzyme Q₁₀ was dissolved in ethanol, adsorbed onto microcrystalline cellulose and dried under reduced pressure. This was mixed with cornstarch, lactose, carboxymethylcellulose and magnesium stearate under a nitrogen atmosphere, then an aqueous solution of polyvinylpyrrolidone was added as a binder and the mixture was granulated by a conventional method. This was mixed with talc as a lubricant and the mixture was tableted. The tablets were packed under a nitrogen atmosphere and refrigerated.

| | |
|---|---|
| oxidized coenzyme Q₁₀ | 20 parts by weight |
| citric acid | 10 parts by weight |
| cornstarch | 25 parts by weight |
| lactose | 15 parts by weight |
| calcium carboxymethylcellulose | 10 parts by weight |
| microcrystalline cellulose | 40 parts by weight |
| polyvinylpyrrolidone | 5 parts by weight |
| magnesium stearate | 3 parts by weight |
| talc | 10 parts by weight |

### (Formulation Example 11) (tablet)

Reduced coenzyme Q₁₀ was dissolved in ethanol, adsorbed onto microcrystalline cellulose and dried under reduced pressure. This was mixed with cornstarch, lactose, carboxymethylcellulose and magnesium stearate under a nitrogen atmosphere, then an aqueous solution of polyvinylpyrrolidone was added as a binder and the mixture was granulated by a conventional method. This was mixed with talc as a lubricant and the mixture was tableted. The tablets were packed under a nitrogen atmosphere and refrigerated.

| | |
|---|---|
| reduced coenzyme Q₁₀ | 20 parts by weight |
| citric acid | 10 parts by weight |
| cornstarch | 25 parts by weight |
| lactose | 15 parts by weight |
| calcium carboxymethylcellulose | 10 parts by weight |
| microcrystalline cellulose | 40 parts by weight |
| polyvinylpyrrolidone | 5 parts by weight |
| magnesium stearate | 3 parts by weight |
| talc | 10 parts by weight |

### (Formulation Example 12) (tablet)

Reduced coenzyme Q₁₀ and oxidized coenzyme Q₁₀ were dissolved in ethanol, adsorbed onto microcrystalline cellulose and dried under reduced pressure. This was mixed with cornstarch, lactose, carboxymethylcellulose and magnesium stearate under a nitrogen atmosphere, then an aqueous solution of polyvinylpyrrolidone was added as a binder and the mixture was granulated by a conventional method. This was mixed with talc as a lubricant and the mixture was tableted. The tablets were packed under a nitrogen atmosphere and refrigerated.

| | |
|---|---|
| reduced coenzyme Q₁₀ | 20 parts by weight |
| oxidized coenzyme Q₁₀ | 0.4 part by weight |
| citric acid | 10 parts by weight |
| cornstarch | 25 parts by weight |
| lactose | 15 parts by weight |
| calcium carboxymethylcellulose | 10 parts by weight |
| microcrystalline cellulose | 40 parts by weight |
| polyvinylpyrrolidone | 5 parts by weight |
| magnesium stearate | 3 parts by weight |
| talc | 10 parts by weight |

### (Formulation Example 13) (drink)

Oxidized coenzyme Q₁₀, cyclodextrin and vitamin E were mixed, powderized by drying under reduced pressure, and dispersed in water by a conventional method to give a drink.

| | |
|---|---|
| oxidized coenzyme Q₁₀ | 50 parts by weight |
| cyclodextrin | 200 parts by weight |
| vitamin E | 30 parts by weight |

### (Formulation Example 14) (drink)

Reduced coenzyme Q₁₀, cyclodextrin and vitamin C were mixed, powderized by drying under reduced pressure, and dispersed in water by a conventional method to give a drink.

| | |
|---|---|
| reduced coenzyme Q₁₀ | 50 parts by weight |
| cyclodextrin | 200 parts by weight |
| vitamin C | 30 parts by weight |

### (Formulation Example 15) (drink)

Reduced coenzyme Q₁₀, oxidized coenzyme Q₁₀, cyclodextrin and vitamin C were mixed, powderized by drying under reduced pressure, and dispersed in water by a conventional method to give a drink.

| | |
|---|---|
| reduced coenzyme Q₁₀ | 49 parts by weight |
| oxidized coenzyme Q₁₀ | 1 part by weight |
| cyclodextrin | 200 parts by weight |
| vitamin C | 30 parts by weight |

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

The present invention is based on JP 2006-016274 filed in Japan, which all the content is encompassed in the present specification.

## Claims

1. A composition for normalizing the blood pressure, comprising reduced coenzyme Q represented by the following formula (1): 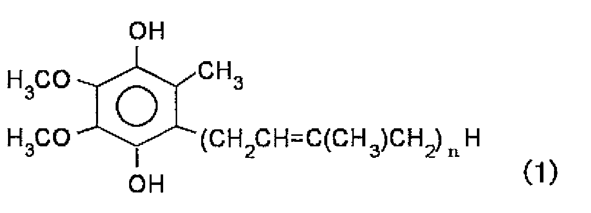 wherein n is an integer of 1 - 12, as an active ingredient.

2. The composition of claim 1, further comprising oxidized coenzyme Q represented by the following formula (2): 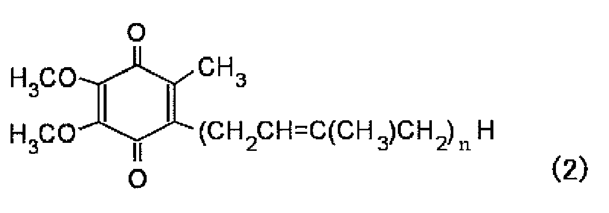 wherein n is an integer of 1 - 12.

3. The composition of claim 1 or 2, which improves hypertension.

4. The composition of claim 1 or 2, which improves hypotension.

5. A composition for normalizing the blood pressure, comprising oxidized coenzyme Q represented by the following formula (2): 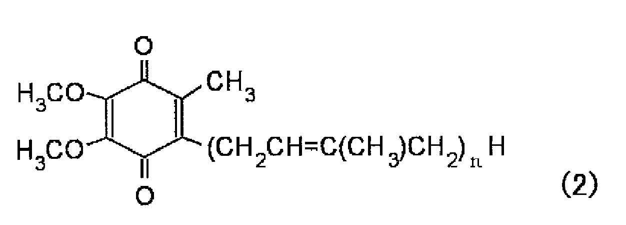 wherein n is an integer of 1 - 12, as an active ingredient, which improves hypotension.

6. The composition of any one of claims 1 to 4, wherein the reduced coenzyme Q is a reduced coenzyme Q₁₀ wherein n is 10.

7. The composition of any one of claims 2 to 5, wherein the oxidized coenzyme Q is an oxidized coenzyme Q₁₀ wherein n is 10.

8. The composition of any one of claims 1 to 7, comprising an antioxidant substance and/or an antioxidant enzyme.

9. The composition of claim 8, wherein the antioxidant substance is at least one kind selected from the group consisting of vitamin E, vitamin E derivative, vitamin C, vitamin C derivative, probucol, lycopene, vitamin A, carotenoids, vitamin B, vitamin B derivative, flavonoids, polyphenols, glutathione, α-lipoic acid and selenium.

10. The composition of claim 8, wherein the antioxidant enzyme is at least one kind selected from the group consisting of superoxide dismutase (SOD), glutathione peroxidase, glutathione-S-transferase, glutathionereductase, catalase and ascorbic acid peroxidase.

11. The composition of any one of claims 1 to 8, comprising a nutritious supplement component.

12. The composition of claim 11, wherein the nutritious supplement component is at least one kind selected from the group consisting of amino acid, metal ion, saccharides, proteins, fatty acids and vitamins.

13. The composition of any one of claims 1 to 12, which is a drink or food.

14. The composition of any one of claims 1 to 12, which is an oral pharmaceutical product.

15. The composition of any one of claims 1 to 12, which is a feed or pet food.

16. A method of normalizing the blood pressure, which comprises administering a composition comprising reduced coenzyme Q represented by the following formula (1): 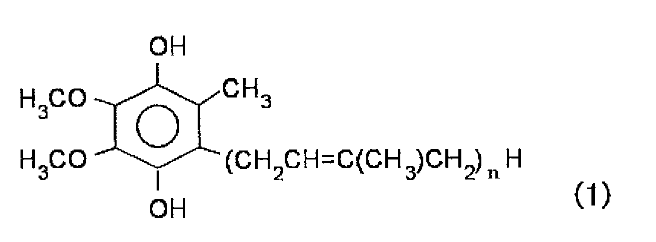 wherein n is an integer of 1 - 12, as an active ingredient, to a subject of administration.

17. The method of normalizing the blood pressure, which comprises administering the composition of claim 16 further comprising oxidized coenzyme Q represented by the following formula (2): 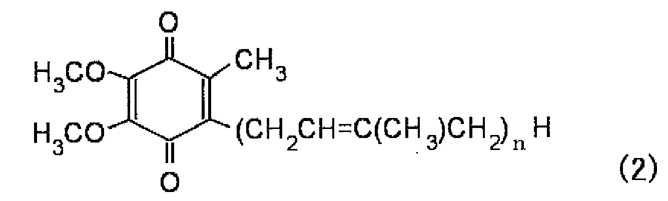 wherein n is an integer of 1 - 12, to a subject of administration.

18. The method of claim 16 or 17, wherein the normalization of blood pressure is an improvement of hypertension.

19. The method of claim 16 or 17, wherein the normalization of blood pressure is an improvement of hypotension.

20. A method of improving hypotension, which comprises administering a composition comprising oxidized coenzyme Q represented by the following formula (2): 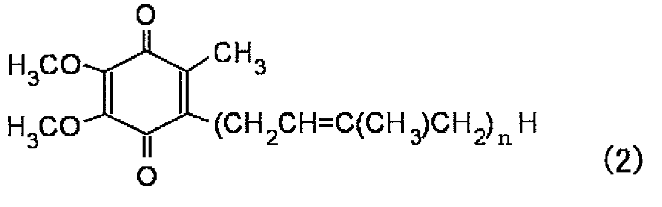 wherein n is an integer of 1 - 12, as an active ingredient, to a subject of administration.

21. Use of reduced coenzyme Q represented by the following formula (1): 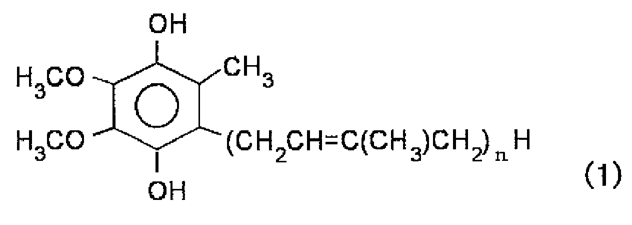 wherein n is an integer of 1 - 12, for the production of a composition for normalizing the blood pressure.

22. The use of claim 21, further comprising use of oxidized coenzyme Q represented by the following formula (2): 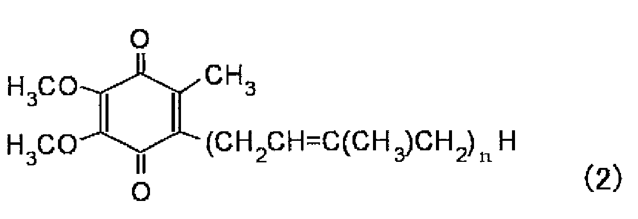 wherein n is an integer of 1 - 12.

23. The use of claim 21 or 22, wherein the blood pressure normalizing composition is a composition for improving hypertension.

24. The use of claim 21 or 22, wherein the blood pressure normalizing composition is a composition for improving hypotension.

25. Use of oxidized coenzyme Q represented by the following formula (2): 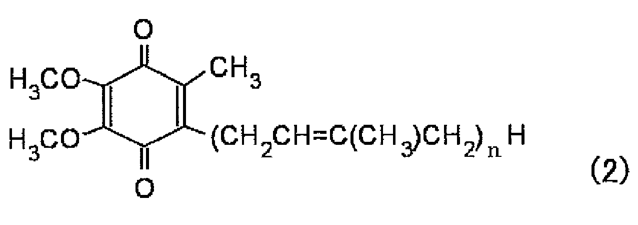 wherein n is an integer of 1 - 12, for the production of a composition for improving hypotension.

26. A commercial package comprising the composition of any one of claims 1 to 15 and a written matter relating to the composition, which states that the composition can or should be used for normalizing the blood pressure.
